# EUROPÄISCHE PATENTSCHRIFT

(11) **EP 2 347 407 B1**
(45) Veröffentlichungstag und Bekanntmachung des Hinweises auf die Patenterteilung: **20.05.2015**
(21) Anmeldenummer: 08874925.4
(22) Anmeldetag: 18.10.2008
(51) Int. Cl.: G10K 15/06, A61H 31/00

(54) **IMPULSWELLENGENERATOR**
PULSE WAVE GENERATOR
GÉNÉRATEUR D'ONDES D'IMPULSIONS

(43) Veröffentlichungstag der Anmeldung: 27.07.2011
(73) Patentinhaber: Weth, Gosbert, 97688 Bad Kissingen (DE)
(72) Erfinder: Weth, Gosbert, 97688 Bad Kissingen (DE)
(86) Internationale Anmeldenummer: PCT/DE2008/001707
(87) Internationale Veröffentlichungsnummer: WO 2010/043190

(56) Entgegenhaltungen:
- EP-A- 0 450 364
- WO-A-2005/018469
- DE-A1- 19 605 777
- DE-A1-102006 024 701

## Beschreibung

Die Erfindung bezieht sich auf einen Impulswellengenerator bestehend aus einem Gehäuse mit einem Druckfeldgenerator, von dem einzeln ansteuerbare Druckimpulses abstrahlbar sind und einer Druckimpulsfokirssierung, mittels derer die Druckimpulse in einem Fokussierungspunkt in oder an einem Körper des Lebewesens fokussierbar sind und einem Druckimpulskoppler, von dem die Druckimpulse auf den Körper übertragbar sind und einer Steuerung für die Intensität und den Zeitpunkt jedes Druckimpulses, wobei wenigstens ein weiterer Feldgenerator integriert ist, durch den einzeln ansteuerbare Feldimpulse im Fokussierungspunkt erzeugbar sind und die Intensität und der Zeitpunkt jedes Magnetfeldimpulses steuerbar ist.

Auf aktuellem Stand der Technik ist die Einbringung von Impulsen in den Körper von Lebewesen als therapierende Maßnahme weithin bekannt. Ein Beispiel ist die so genannte Atlastherapie, die auf den französischen Arzt Arlen zurückgeht (siehe "manuelle Medizin", 27. Jahrgang, Heft 4, Auflage 1989, Seite 82 Springerverlag). Bei der Atlastherapie wird durch manuelle Impulse, insbesondere leichte Schläge mit dem Finger auf einem der Querfortsätze des ersten Halswirbels Schmerzen im Wirbelsäulenbereich therapiert. Außerdem können Verspannungen im Bereich der Wirbelsäule reduziert oder sogar aufgelöst werden. Sogar erste Folgeerscheinungen der Multiplen Sklerose können gebessert werden, sodass z. B. eine akute Rückbildung von Lähmungen möglich ist.

Ein wesentlicher Nachteil ist, dass die Bemessung der Impulse sehr viel Erfahrung und Routine vom ausübenden Arzt verlangt.

Ein anderes, bekanntes Beispiel für die segensreiche Einwirkung von schockartigen Impulsen ist der Lithotripter, der zur Zertrümmerung von Nieren- und Gallensteinen dient. Er besteht aus einem Druckfeldgenerator, der einzelne Druckimpulse abstrahlt, die sich wie Schallwellen verhalten und deshalb in einer akustischen Linse auf einen Fokussierungspunkt im Körper des zu behandelnden Patienten fokussiert werden. Dabei überträgt ein Druckimpulskoppler, auch Akustikkoppler genannt, die Druckimpulse vom Lithotripter auf den Körper des Patienten. Dieser Koppler besteht meist aus einem flüssigkeitsgefüllten, dauerhaft flexiblen Beutel, der sich an einer Seite zur Akustiklinse -hier Druckimpulsfokussierung genannt - hin öffnet und an deren äußerem Rand befestigt ist.

Die Druckimpulsfokussierung und der Druckimpulskoppler sorgen dafür, dass der größte Teil der mechanischen Energie im Fokussierungspunkt gebündelt wird. Der Lithotripter wird mit Hilfe eines Ultraschalldiagnosegerätes oder eines Röntgengerätes so ausgerichtet, dass sich der Fokussierungspunkt in einem Gallenstein oder Nierenstein befindet. Wenn die Ausrichtung des Gerätes fixiert ist, wird die Intensität und die Frequenz der Druckimpulswellen langsam gesteigert und der Nieren- oder Gallenstein sukzessive in immer kleinere Bruchstücke zerteilt, bis er im Idealfall pulverisiert ist. Auf dem Bildschirm des Diagnosegerätes lässt sich verfolgen, wie der eingangs feste Block allmählich in eine diffuse Wolke aufgelöst wird.

Der Lithotripter kann zwar die Steine allmählich zertrümmern, kann sie aber nicht abtransportieren. Diese finale Tätigkeit übernimmt der Körper selber, in dem er die pulverisierten Bruchstücke zusammen mit dem Harn auf "normalem Wege" abtransportiert.

Der Lithotripter ist also ein sehr eingängiges Beispiel dafür, dass Schockwellen, die auf einen bestimmten, begrenzten Bereich des menschlichen Körpers einwirken und dort auf eine stationäre, unwillkommene und deshalb als krank eingestufte Konfiguration stoßen, die stationäre Ordnung in diesem Bereich durcheinander bringen, wie z. B. das Auflösen der Steine, und erst dadurch den körpereigenen Systemen einen Zugriff ermöglichen, sodass sie nunmehr die ursprüngliche Ordnung wiederherstellen können.

Der Lithotripter ist ein prägnantes Beispiel für die Erzeugung eines mechanischen Feldes durch einen Druckfeldgenerator.

Es sind jedoch in der aktuellen Medizintechnik auch Geräte bekannt, die als Therapie ein schockartiges, elektrisches Feld auf den Ort der Beschwerden aufbringen. Ein Beispiel dafür ist der Defibrilator. Er besteht aus einem Kondensator, der schlagartig entladen wird, sodass über zwei Elektroden auf der menschlichen Brust kurzzeitig ein sehr hohes Spannungspotential - bis 750 Volt - an den Körper angelegt wird und deshalb über den Körper ein Strom fließt, dessen Stärke von dem jeweiligen Körperwiderstand abhängt, der im Mittel meist 50 Ohm beträgt, sodass sich ein Strom von bis zu 15 Ampere einstellt. Weil der Kondensator nur eine begrenzte Energiemenge von typischerweise 150 Joule speichert, sinken Spannung und Stromstärke sehr schnell ab, sodass keine bleibenden Schäden an anderen, eigentlich nicht betroffenen Körperregionen und Körperteilen sowie am Herzen selber verursacht werden.

Der Defibrilator wird vor allem beim so genannten Kammerflimmern eingesetzt, der Ursache für etwa 85 % aller plötzlichen Herztode, bei dem durch eine elektrisch kreisende Erregung in den Myokardzellen, den Zellen des Herzens, dessen ordnungsgemäßes Schlagen blockiert wird. Die elektrische Impulswelle des Defibrilators depolarisiert eine große Anzahl von Zellen gleichzeitig, sodass sie für eine relativ lange Zeit nicht mehr erregbar sind. Diese "Refraktärzeit" beträgt üblicherweise etwa 250 Millisekunden. Dann kann das stillstehende Herz durch eine Herz-Lungenmassage wieder zum Schlagen mit seiner normalen Frequenz angeregt werden.

Beiden Verfahren gemeinsam ist, dass die schockartige Impulswelle einen höchst unerwünschten und stationären Zustand zuerst einmal "nur" maßgeblich verändert. Auch dieser neue Zustand ist noch nicht der eigentlich gewünschte, aber er hat den ganz entscheidenden Vorteil, dass nunmehr wieder körpereigene Reparatur- und Rettungsabläufe auf den betroffenen Bereich zugreifen können und die Rückkehr in einen wünschenswerten, normalen Zustand ermöglichen. In stark verkürzter Beschreibung können die Impulswellen auch als "Hilfe zur Selbsthilfe" eingestuft werden.

Zu den Vorzügen dieses Prinzips zählt, dass nur relativ kurze und kostengünstige Vorbereitungen für den Patienten erforderlich sind und die Kosten des Gerätes bei mehrfacher Anwendung bezogen auf den einzelnen Anwendungsfall sehr niedrig sind. Der wichtigste Vorzug ist, dass der Eingriff keine Nebenwirkungen oder Kollateralschäden verursacht.

So ist z. B. die Entfernung von Nieren- oder Gallensteinen auch operativ möglich. Die dafür erforderliche Verwundung des Patienten und der wochenlange Heilungsprozess stehen jedoch in ganz dramatischem Gegensatz zur Zeitdauer einer Nierensteinzertrümmerung. Dementsprechend sind auch die Kostenunterschiede für die Behandlung dramatisch.

Ebenso sind für Eingriffe am Herzen operative Eingriffe nicht nur bekannt sondern auch üblich, nicht jedoch bei Kammernflimmern, da der Patient auch bei schnellstmöglicher Einleitung der Operation bereits nicht mehr am Leben wäre.

Der wesentliche Vorteil der hier exemplarisch vorgestellten Behandlungen von inneren Organen und anderen räumlich begrenzten, von einer Störung heimgesuchten Bereichen des Körpers ist also, dass die Behandlung durch angrenzende Organe und Körperstrukturen hindurch zerstörungsfrei und nebenwirkungsfrei nur die eigentlich beabsichtigte Wirkung entfaltet.

Ein wesentlicher Nachteil ist, dass die Geräte nur für eine ganz bestimmte Krankheit oder einen ganz bestimmten Problemfall konfiguriert und verwendbar sind. So beschreibt z. B. die EP 0 301 360, Franz Grasser, einen Lithotripter, der jedoch nur für Gallensteine optimiert ist. Es wird nicht einmal eine Anwendbarkeit für Nierensteine, geschweige denn für andere unerwünschte Ansammlungen oder Konzentrationen im Körper genannt.

Die PS US 5,119,832, Ravi Xavier, beschreibt einen Epiduralkatheter, der zwischen zwei Wirbel des Rückgrades bis in den Periduralraum, also ganz nahe an das Rückenmark, heran geschoben wird. Zusätzlich zu einem Betäubungsmittel werden über außen angebrachte Elektroden elektrische Impulse mit einer Spannung von bis zu 10 Volt und einer Frequenz von bis zu 120 Herz in den Bereich des Rückenmarks abgegeben. Diese stoßweise auftretenden elektrischen Felder dämpfen die Schmerzempfindungen eines Patienten deutlich, sodass die erforderliche Dosis von Schmerzmitteln deutlich reduziert werden kann. Die Betäubungswirkung reicht für größere Operationen und Eingriffe wie z. B. Kaiserschnitte aus. Dieses Beispiel zeigt eine weitere Anwendungsmöglichkeit für Impulswellen eines elektrischen Feldes.

Ein weiteres, bekanntes Beispiel für Impulswellen eines mechanischen Kraftfeldes gibt die PS US 3,396,721, Mencacci, wieder. Hier wird ein Stößel durch einen Elektromagnet linear bewegt, sodass er 5-15 Mal pro Minute bewegt wird. Diese mechanischen Schläge werden von Außen auf die Wirbelsäule im Bereich der Lendenwirbel aufgebracht und sorgen für eine Aktivierung der Peristaltik, indem zusätzliche Bewegungsimpulse an das Nervensystem der Muskulatur des Darms ausgelöst werden.

Ein weiteres, bekanntes Beispiel für die Anwendung von Impulswellen eines mechanischen Feldes nennt die PS US 3,861,383, Kovach, mit einem Hautbehandlungs- und Hautmassagegerät, das mit einer Frequenz von ca. 30 - 55 Herz Flüssigkeit aus einer Düse pulsierend auf die Haut austreten lässt, wobei der Einwirkungsbereich durch einen Zylinder begrenzt wird, der auf die Haut aufgesetzt ist. Durch die Anregung der Durchblutung wird der Alterungsprozess der Haut verlangsamt und die Bildung von Falten verzögert. Ein Nachteil des Gerätes ist die große austretende Wassermenge, die aufgefangen oder abgeführt werden muss. Deshalb bedarf die Anwendung längerer Vorbereitungen und Nachbereitungen. Eine weitere Einschränkung ist, dass das Gerät nur für die Haut, also direkt für die Oberfläche des Körpers geeignet ist, andere Anwendungen sind ausgeschlossen.

Ein weiteres, bekanntes Beispiel für einen Impulswellengenerator, der sich eines mechanischen Feldes bedient, nennt die PS US 4,265,228 Paul M Zoll. Sie beschreibt ein pistolenartiges Gehäuse, in dessen Lauf eine Masse von einem Elektromagneten beschleunigt wird, bis sie aus dem Lauf etwas heraustritt. Die Öffnung des Laufs wird auf die Brust des Patienten gesetzt und dann die Masse mit einer vorgewählten Frequenz und einer vorgewählten Intensität beschleunigt, bis sie auf der Brust auftrifft und dann wieder von einer Feder in die Ausgangsposition zurückgeholt wird.

Der Vorteil dieses Gerätes ist, dass Frequenz und Aufprallintensität im Vergleich zu einer manuellen Druckmassage mit erheblich höherer Genauigkeit vorgegeben wird, sodass die Impulse einerseits für die gewünschte Beeinflussung des Herzschlages ausreichen, andererseits aber nicht so groß sind, als dass z. B. Rippen gebrochen werden könnten. Vorteilhaft ist, dass das Gerät in seinem Funktionsprinzip einem bekannten Nagelapparat ähnelt und deshalb zu vergleichsweise sehr niedrigen Kosten herstellbar und deshalb leicht verfügbar ist.

Als Nebenwirkung wird bei längerem Gebrauch eine gewisse Hautrötung beobachtet, die aber relativ schnell wieder zurückgebildet wird. Der entscheidende Nachteil ist jedoch, dass außer der Anwendung als Herzschrittmacher keine weiteren Anwendungen belegt sind.

Die vorgenannten, bekannten Beispiele auf aktuellem Stand der Technik zeigen, dass stoßartig einwirkende Impulse eines mechanischen oder eines elektrischen Feldes in einem bestimmten, begrenzten Bereich des Körpers eines Lebewesens indirekt heilend wirken können, wenn die Art ihres Feldes - mechanisch oder elektrischund die Intensität und der Zeitpunkt ihres jeweiligen Impulses recht genau auf die zu behandelnde Störung abgestimmt sind.

Des Weiteren ist nach aktuellem Stand der Technik auch die Anwendung von Impulsen eines Magnetfeldes in der Medizintechnik bekannt. Die PS US 1,962,565, Lakhovsky, beschreibt eine Spule, die auf den Körper gerichtet oder gelegt wird und von einer getakteten elektrischen Energiequelle und einen Kondensator mit Stromimpulsen versorgt wird.

Die PS US 5,556,418, Pappas beschreibt ebenfalls eine Stromdurchflossene Spule auf dem Körper des Patienten. Die Stromimpulse zur Versorgung dieser Spule werden mit noch steileren Flanken angesteuert, sodass noch höhere magnetische Feldstärken im Körper des Patienten entstehen. Auch für diese Anordnung ist nachgewiesen, dass Impulswellen durch die Änderung der Feldstärke heilend wirken, wenn sie jeweils innerhalb eines engen Bereiches an den zu behandelnden Defekt angepasst sind.

Das EP 0 683 657 des Erfinders erläutert eine Quelle "impulsartiger Wellen", die auf die Behandlung von Schmerzzuständen oder des vegetativen Nervensystems eingeschränkt ist. Ausführlich beschrieben wird das Erzeugen einer Druckimpulses und damit erreichbare, schmerzlindernde oder schmerzstillende Effekte. Alternativ zum Druckimpuls kann auch ein elektrischer Impuls oder eine elektromagnetische Welle zur Schmerztherapie verabreicht werden. Dadurch wird eine klinische Wirkung gezeigt.

Die wesentliche Einschränkung dieses Patentes ist, dass das Gerät nur für die Behandlung von Schmerzen und Schmerzzuständen geeignet ist und damit in der Regel nur die Symptome einer Krankheit, nicht aber deren Ursache bekämpft, von den relativ wenigen Fällen z.B. eines Phantomschmerzes abgesehen.

Die Offenlegungsschrift DE 10 2006 024 701 stellt eine Kombination aus einer mechanischen und aus einer elektrischen Impulsquelle vor, die beide auf das Herz einwirken. Insbesondere die Reanimation eines nicht mehr schlagenden Herzens ist ausführlich beschrieben, Neben dem Herzen wird auch ganz pauschal die Möglichkeit der Einwirkung auf andere Organe erwähnt.

Auf aktuellem Stand der Technik sind also Geräte zur Behandlung eines Patienten entweder mit mechanischen oder mit elektrischen oder mit magnetischen Impulsen bekannt. Ebenfalls bekannt ist ein Gerät, das mechanische und elektrische Impulse kombiniert.

Auf diesem Hintergrund hat sich die Erfindung die Aufgabe gestellt, ein Gerät zur Behandlung eines begrenzten Körperbereiches mit einer Kombination impulsartiger Wellen zu entwickeln, das bei möglichst zahlreichen Organen und an möglichst vielen anderen Stellen des Körpers einsetzbar ist und auf möglichst viele verschiedene Erkrankungen, insbesondere auch auf Krebs und auf möglichst viele Schmerzarten und schmerzende Orte eingestellt werden kann, ohne dabei andere Bereiche zu beeinflussen oder gar zu schädigen.

Als Lösung präsentiert die Erfindung, dass als Feldgenerator zur Erzeugung eines Magnetfeldes eine Spule dient, die durch die Entladung eines auf bis zu 50 kV aufgeladenen Kondensators über eine Plasmakammer oder über eine Funkenstrecke von einem sehr hohen Stromimpuls von bis zu 3 kA durchflossen wird, woraus sich ein elektromagnetischer Impuls ergibt, dessen Energieinhalt kurzzeitig sehr hoch ist und der durch Induktion das effektive Transmembranpotential einer Tumorzelle kurzzeitig auf einen normalen Wert anhebt.

Es ist also ein ganz wesentlicher Grundgedanke der Erfindung, dass zur Einwirkung auf den zu therapierenden Bereich des Körpers ein mechanisches Feld und ein magnetisches Feld gewählt werden und beide Felder impulsartig auf den betroffenen Bereich einwirken. Dabei ist es ein weiteres ganz wichtiges Merkmal, dass beide Feldarten auf einen gemeinsamen Fokussierungspunkt ausgerichtet werden, also in anderen, nicht betroffenen Bereichen überhaupt nicht zur Wirkung kommen.

Dazu gehört auch, dass bei tiefer im Körper gelegenen Bereichen die Strecke von der Außenfläche des Körpers bis zum Fokussierungspunkt ebenfalls ohne Nebenwirkungen von den einwirkenden Feldern überwunden werden kann. Die beiden einwirkenden Felder, nämlich ein mechanisches und ein magnetisches werden also nicht nur additiv überlagert, sondern beide auf einen ganz bestimmten Fokussierungspunkt ausgerichtet. Dadurch konzentriert sich ihre Energie in diesem Punkt, was die heilende Wirkung erhöht und bleibt von anderen Bereichen fern, was jegliche Wirkung auf andere Bereiche so weit reduziert, dass keine dauerhafte Wirkung verbleibt.

Ein erfindungsgemäßer Impulswellengenerator besteht also grundsätzlich aus zwei Feldgeneratoren, die jeweils einen Feldimpuls erzeugen, der auf den Fokussierungspunkt wirkt wird und dessen Intensität und dessen Zeitpunkt steuerbar ist.

Der erste der beiden Feldgeneratoren ist stets ein Druckfeldgenerator, dessen Druckimpulse von einer Druckimpulsfokussierung in den Fokussierungspunkt im oder am Körper des Lebewesens fokussiert wird. Zur Übertragung von dem Impulswellengenerator auf den Körper dient ein Druckimpulskoppler und zur Steuerung der Intensität und des Zeitpunktes der Impulse der beiden Feldgeneratoren eine Steuerung.

Ein erfindungsgemäßer Impulswellengenerator weist neben dem zuvor beschriebenen Druckfeldgenerator einen weiteren Feldgenerator auf, der als Magnetfeldgenerator ausgeführt ist. Er ist eine Spule, die durch die Entladung eines auf eine sehr hohe Spannung aufgeladenen Kondensators über eine Plasmakammer oder eine Funkenstrecke von einem sehr hohen Stromimpuls durchflossen wird. In der Praxis liegen am Kondensator Spannungen bis zu 50 kV an. Damit werden Stromimpulse in der Spule von bis zu 3 kA erzeugt.

Daraus ergibt sich ein elektromagnetischer Impuls, dessen Energieinhalt kurzzeitig sehr hoch ist, sodass er im Körper entsprechende Barrieren überwinden kann. Ein hoch interessantes Beispiel dafür ist das so genannte Transmembranpotential einer Zelle. Es beträgt bei gesunden Zellen etwa 80 bis 100 mV, bei stark gereizten Zellen, z.B. durch chronischen Schmerz, etwa 60 - 80 mV, bei Tumorzellen jedoch nur 15 bis 20 mV.

Der hohe Wert des Transmembranpotentials einer gesunden Zelle entspricht bei den extrem geringen Abmessungen der Zelle in der Größenordnung von einigen Angström einem Spannungsgefälle von etwa 1.000 kV pro Meter. Diese hohe Zahl plausibilisiert die Unempfindlichkeit der gesunden menschlichen Zellen gegenüber sehr hohen elektrischen Potentialen, die auf sie einwirken.

Es wird angenommen, dass das vorerwähnte elektromagnetische Wechselfeld durch Induktion das effektive Transmembranpotential der Tumorzelle kurzzeitig auf einen normalen Wert anhebt und dadurch normalen Reparaturmechanismen des Körpers einen Zugang ermöglicht.

Diese Fähigkeit unterscheidet das erfindungsgemäße Gerät und damit durchgeführte Therapien grundlegend von den meisten bisher bekannt gewordenen Vorrichtungen und Behandlungen von Carcinomen, die nur das Innere der Zelle und deren DNA beeinflussen. Ein erfindungsgemäßer Impulswellengenerator beeinflusst hingegen auch die Zellmembrane und die sie umgebende Zellmatrix.

Tumorzellen sind als Teil ihrer Zellmatrix mit einem Schutzmantel aus einer Hyaluron-Fibrin-Schicht umgeben, der z.B. Makrophagen von einem Zugriff auf die Tumorzelle abzuhalten scheint, indem er einen ordnungsgemäßen, körperlichen Heilungsprozess vorzutäuschen scheint.

Der Zweite Feldgenerator kann entweder ein magnetisches oder ein elektrisches oder ein elektromagnetisches Feld erzeugen.

Diese drei möglichen Ausführungsformen sind nicht damit zu verwechseln, dass jeder Stromdurchflossene Leiter um sich herum ein magnetisches Feld aufbaut, dass also bei der Erzeugung des Feldes innerhalb des Gerätes grundsätzlich beides wirksam wird Entscheidend ist vielmehr, welche Feldart nach außen hin aus dem Impulswellengenerator heraus und in den Körper des zu behandelnden Patienten hinein wirksam wird.

Hier ist zu unterscheiden, dass entweder nur ein magnetisches Feld oder nur ein elektrisches oder ein elektromagnetisches Feld wirksam wird. Ein reines magnetisches Feld entsteht z. B. zwischen den beiden Polen eines Hufeisenmagneten. Es ist also durchaus denkbar, dass in unmittelbarer Nachbarschaft des Fokussierungspunktes F die beiden Schenkel eines U-förmigen Blechpaketes von einem Elektromagneten angeordnet werden. Eine andere bekannte Ausführungsform ist ein Ringmagnet, der um den Körper des Patienten herum aufgebaut wird.

Ein elektrisches Feld wird z. B. durch zwei voneinander beabstandete Elektroden erzeugt, wobei etwa in deren Mitte der Fokussierungspunkt F anzuordnen ist. An diese beiden Elektroden ist dann jeweils eine elektrische Spannung anzulegen.

Ein elektromagnetisches Feld entsteht dann, wenn eine Spule mit hochfrequentem Wechselstrom durchflossen wird oder wenn ein Stromimpuls mit sehr steilen Flanken hochfrequente Anteile generiert. Dadurch bildet sich eine elektromagnetische Welle, die sich unabhängig von Leitern durch den Raum hinweg fortpflanzt.

Im einfachsten Fall geben beide Feldgeneratoren einen Impuls ab, der Druckfeldgenerator also einen Druckimpuls und z. B. ein elektromagnetischer Feldgenerator einen Elektromagnetimpuls. Sehr gute Behandlungserfolge wurden bei den verschiedensten Anwendungen erreicht, wenn ein Druckimpuls von bis zu 100 Millisekunden Dauer mit einem elektromagnetischen Impuls von maximal der gleichen Länge kombiniert wurde.

Ein Druckimpuls, der auf die Umgebungsluft wirkt, wird als Schall empfunden. Deshalb ist eine interessante Ausführungsform des Druckfeldgenerators eine Schallquelle. Eine Fokussierung einer Schallwelle wird auch als mechanische Linse oder Akustiklinse bezeichnet. Durch einen Akustikkoppler oder Koppelsack - einen mit Flüssigkeit befüllten, flexiblen Beutel - werden die als Schallwellen hörbaren Druckimpulse auf den Körper des Patienten übertragen. Wenn gleichzeitig ein elektromagnetisches Feld im Fokussierungspunkt wirksam ist, können damit Tumore, Tromben und viele andere Krankheiten erfolgreich therapiert werden, was durch zahlreiche Behandlungserfolge nachweisbar ist.

Dabei ist es stets erforderlich, den Ort des Fokussierungspunktes, die Intensität und den Zeitpunkt jedes Impulses an die zu behandelnden Körperregionen und die dort wirkende Störung innerhalb sehr enger Grenzen anzupassen. Mit dem erfindungsgemäßen Impulswellengenerator sind erfolgreich Krankheiten und Störungen zu behandeln, deren Entstehung nach heutigem Wissensstand nicht befriedigend erklärt werden können, wie z. B. Krebs.

Deshalb kann - derzeit - nicht bei jeder mit dem erfindungsgemäßen Impulswellengenerator erfolgreich behandelter Krankheit auch eine genaue Erklärung der Wirkung abgegeben werden. Vielmehr muss der Anwender in zahlreichen Versuchen herausfinden, wie groß die Anzahl der Impulse, ihre Stärke und ihr zeitlicher Abstand für jede Behandlung ist und in welchem Abstand und wie oft diese Behandlung wiederholt werden muss. Für die Kombination aus einem Druckfeldgenerator mit einem elektromagnetischen Feldgenerator liegen jedoch zahlreiche, beeindruckende Behandlungsergebnisse für die verschiedensten Krankheiten und Störungen vor, die im Anschluss an die Beschreibung der möglichen Gerätevarianten vorgestellt werden.

Es ist nämlich das Ziel dieser Erfindung, ein universell anwendbares Behandlungsgerät mit Impulswellen zu schaffen, das für mehrere Krankheiten anwendbar ist und die Vorteile einer schnell einsetzbaren, unblutigen und nebenwirkungsfreien Behandlungsmethode nutzbar macht.

Es ist Aufgabe des Anwenders, aus einer genauen Kenntnis der ordnungsgemäßen Funktion und der Fehlfunktion des betroffenen Körperbereiches oder des betroffenen Organs heraus abzuleiten, welche Feldimpulse zur Behandlung eingesetzt werden. Mangels dieser Kenntnisse dürfte das jedoch vergleichsweise selten der Fall sein. Weitaus häufiger werden die jeweiligen Behandlungsparameter experimentell ermittelt werden, indem sich der Anwender mit Versuchsreihen an eine optimale Wirkung herantastet. Diese Methode ist insofern ethisch voll verantwortbar, als dem Patienten andere, bisher bekannte, konventionelle Behandlungsmethoden in vollem Umfang offen bleiben und durch eine zusätzliche Behandlung mit dem Impulswellengenerator keine Nebenwirkungen entstehen.

Da bei einem solchen Vorgehen Wechselwirkungen mit eventuellen Medikamentierungen und andern Behandlungsarten denkbar sind, ist natürlich die ausschließliche Behandlung mit dem Impulswellengenerator vorzuziehen, da nur dann ganz klar feststeht, dass ein Behandlungserfolg ausschließlich auf die Anwendung des Impulswellengenerators zurückzuführen ist.

Da es zur erfindungsgemäßen Aufgabe zählt, dem Behandelnden möglichst zahlreiche Behandlungsparameter zur Verfügung zu stellen, kann in einer Ausführungsvariante eines erfindungsgemäßen Impulswellengenerators für jedes Feld die Amplitude und/oder die Frequenz über die Einschaltdauer hinweg als ansteigend und/oder abfallend und/oder oszillierend gewählt werden.

Ein weiteres sehr nützliches Hilfsmittel für einen erfindungsgemäßen Impulswellengenerator ist ein Ultraschalldiagnosegerät, dessen Schallkopf in der Längsachse des Druckfeldgenerators angeordnet ist, und zwar so, dass der Schallkopf durch die Druckimpulsfokussierung und durch den Druckimpulskoppler hindurch etwas über die Außenfläche des Druckimpulskopplers hinausragt und auf diese Weise etwas in den Körper des Patienten eindrückbar ist. Zusätzlich ist der Schallkopf um seine Längsachse verschwenkbar.

Dabei ist entscheidend wichtig, dass die Messebene des Schallkopfes durch den Fokussierungspunkt hindurch verläuft. Dann kann der behandelnde Arzt während der Anwendung des erfindungsgemäßen Impulswellengenerators auf dem Bildschirm sehen, ob eine Störung wie z. B Tumorzellen in der Brust, tatsächlich vom Impulswellengenerator erfasst werden.

Ein erfindungsgemäßer Druckfeldgenerator kann in verschiedenen Bauformen ausgeführt werden. Vorteilhaft ist z. B. eine dauernd elastische Membran, die zumindest an mehreren Punkten ihres Umfanges eingespannt ist und in ihrem Zentrum oder nahe dazu durch einen mechanischen Impuls auslenkbar ist, der etwa senkrecht zur Fläche der Membran und auf den Fokussierungspunkt ausgerichtet ist. Derartige Membranen sind u. a. durch Lautsprecher oder Hupen an Kraftfahrzeugen bekannt und bewährt. In den genannten Anwendungsfällen dient ein Elektromagnet zur Bewegung der Membran.

Für einen erfindungsgemäßen Impulswellengenerator kann jedoch alternativ auch ein Piezzokristall, eine vorgespannte Feder, ein pneumatischer oder hydraulischer Druckspeicher mit einem Zylinder, die Entzündung eines explosiven Treibmittels oder das Verschwenken eines Nockens als Antrieb genutzt werden. Alternativ dazu kann der Druckfeldgenerator auch eine in Richtung des Fokussierungspunktes bewegbare Masse sein, die durch einen mechanischen Impuls in diese Richtung ausgelenkt wird.

Konkrete Behandlungserfolge wurden erzielt, wenn die von der bewegbaren Masse abgegebene kinetische Energie wenigstens 5 Millijoule beträgt und eine Geschwindigkeit von wenigstens 3 Meter pro Sekunde erreicht wird. Dann kann eine Masse von weniger als 0,1 Gramm bereits ausreichen.

Im einfachsten Fall ist die bewegbare Masse eine Kugel, die wie ein Geschoss abgefeuert wird. Die zum Abfeuern aus einem Druckfeldgenerator aufgebrauchte Energie wird dann ausschließlich auf die Kugel fokussiert, die Kugel ist also in dieser Ausführungsform zugleich ihre eigene Fokussierungseinrichtung. Wenn die Kugel auf den Körper auftrifft, springt sie wieder zurück, wenn die Elastizität ihrer Oberfläche im Verhältnis zu ihrer Masse zu gering ist. Wenn die Kugel jedoch sehr weich ausgeführt ist, z. B. aus Schaumstoff, wird die beim Aufprall am Körper reflektierte Energie durch Kompression der Kugel in ihr selbst zwischengelagert und dann ebenfalls an den Körper weitergeleitet. Auf diese Weise übernimmt die Kugel die Funktionalität des Druckimpulskopplers mit.

Wenn eine bewegte Kugel auf den Körper eines Patienten auftrifft, so breitet sich der davon ausgelöste Druckimpuls im Körper auch kugelförmig aus. Daraus folgt, dass eine bewegte Masse als Druckfeldgenerator vor allem dann geeignet ist, wenn der gewünschte Fokussierungspunkt recht nahe an der Oberfläche des Patienten liegt.

Eine gewisse Fokussierungswirkung kann dadurch erreicht werden, dass auf dem Festkörper eine Mulde oder eine Vertiefung auf der zum Fokussierungspunkt weisenden Seite der bewegten Masse angeordnet ist. In diesem Fall muss sichergestellt werden, dass diese Seite auch stets auf den Körper auftrifft. Dafür geeignet ist ein Stößel, auf dessen Stirnseite die Mulde oder Vertiefung ausgebildet ist. Die von den Rändern der Mulde jeweils ausgelösten mechanischen Impulse verdichten sich in der Mittelachse der Mulde, wodurch eine Fokussierung erreicht wird. Daraus ergibt sich, dass eine vorteilhafte Ausführungsform für eine bewegte Masse ein Ring ist, der mit einer Flachseite auf den Körper auftrifft, also linear geführt werden muss.

Ein Vorteil des Ringes ist, dass er beim Aufprall in seiner Mitte keine Umgebungsluft komprimiert. Um bei einem Stößel mit einer Mulde auf seiner Stirnseite diesen Nachteil auszugleichen, kann ein Luftkanal vom tiefsten Punkt der Mulde bis an die Außenseite des Stößels eingerichtet werden. Durch diesen Kanal verlässt beim Auftreffen des Stößels und beim Verdichten der Haut ebenfalls komprimierte Luft den Innenraum der Mulde.

Auch für einen mechanisch bewegten Stößel ist es eine vorteilhafte Ankopplung des Druckimpulses, wenn seine Stirnseite von einem dauerhaft flexiblen und mit einer Übertragungsflüssigkeit befüllten Beutel besteht. Der Beutel passt sich beim Aufprall an die Oberfläche des Körpers an und die darin befindliche Flüssigkeit gibt den Druckimpuls mit nur sehr geringen Verlusten an den Körper weiter.

Ein wichtiges Merkmal des erfindungsgemäßen Impulswellengenerators ist die Druckimpulsfokussierung. Dazu dient eine akustische Linse, die aus einem harten und schwingungsbeständigen Material in Form einer Scheibe besteht, deren Rand im Gehäuse so eingespannt ist, dass sie quer zur Richtung des Druckimpulses ausgerichtet ist. Die Scheibe weist auf wenigstens einer Seite eine konkave Vertiefung auf, die rotationssymmetrisch zur Richtung des Druckimpulses geformt ist.

Eine solche Formgebung ist auch aus dem Bereich optisch wirksamer Linsen bekannt. Die Wirkung einer akustischen Linse ist ähnlich, jedoch wird nicht Licht sondern Schall gebündelt.

Ebenso wie eine optische Linse darauf angewiesen ist, dass der auftreffende Lichtstrahl ohne Reflexion in sie eindringen kann, muss auch in einer akustischen Linse der Schall möglichst ungehindert eintreten und austreten können, wofür eine Übertragungsflüssigkeit auf beiden Seiten jeweils vollflächigen Kontakt mit der akustischen Linse hat. Über die Übertragungsflüssigkeit hat die akustische Linse auf der einen Seite Kontakt mit dem Druckimpulskoppler und auf der zweiten Seite mit dem Druckfeldgenerator. Durch die Formgebung ihrer Oberfläche bündelt sie die eintreffenden Druckimpulse im Fokussierungspunkt. Der Querschnitt einer akustischen Linse ebenso wie das Funktionsprinzip ähnelt einer optischen Linse.

Als Druckfeldgenerator wurde bereits eine bewegte Masse genannt. Eine andere Ausführungsform ist eine ebene Membran. Diese Membran kann ebenfalls durch einen mechanischen Impuls ausgelenkt werden, wie zuvor beschrieben. Eine sehr interessante Ausführungsvariante zur Bewegung der Membran ist ein elektrischer Direkt antrieb, mit dem eine besonders gute und feinfühlige Regelung des Druckimpulses möglich ist.

Dazu muss die Membran elektrisch leitfähiges Material wie z. B. Kupfer oder Aluminium enthalten. Mit der zum Körper weisenden Seite grenzt sie an die Übertragungsflüssigkeit im Gehäuse an. Mit der anderen Seite liegt sie auf einer Isolierfolie auf, die ihrerseits wiederum auf einer spiralförmig gewickelten Flachspule aufliegt, die von einem Spulenträger gehalten wird. Über einen Hochspannungsimpulsgenerator kann sie mit Hochspannungsimpulsen beaufschlagt werden. Dadurch erzeugt sie ein elektromagnetisches Feld, welches wiederum im leitfähigen Material der Membran einen Wirbelstrom erzeugt, woraus sich ein weiteres elektromagnetisches Feld ergibt, das dem ursprünglichen Feld entgegen gerichtet ist, wodurch sich die Membran von der Flachspule abstößt.

Da das Feld flächig auf die Membran einwirkt, kann dadurch kurzzeitig ein sehr hoher Schalldruck erzielt werden. In der Praxis wurden mit Druckfeldgeneratoren erfolgreiche Behandlungen erzielt, die Drücke von wenigstens 0,6 bis zu 6 Dyn pro qm erzeugten. Dazu ist ein Hochspannungsimpuls erforderlich, dessen Stromstärke größer als 1 kA ist und dessen Spannung höher als 1 kV ist.

Als weitere, ergänzende Einwirkungsmöglichkeit kann ein erfindungsgemäßer Impulswellengenerator mit einem Apparat zur Infusion flüssiger Wirkstoffe ausgerüstet werden. Damit wird es möglich, die Einwirkungen von Impulswellen zwei verschiedener Feldarten mit einer chemischen Wirkung zu kombinieren.

Ein erfindungsgemäßer Impulswellengenerator kann auf die Behandlung eines sehr weit gespannten Feldes von Beschwerden oder Schmerzempfindungen im oder am Körper von Lebewesen eingerichtet werden, die sich in einem begrenzten Raum lokalisieren.

Dazu zählen Tumore in der Brust, in der Bauchspeicheldrüse, im Gehirn, in der Prostata oder in einem anderen Organ oder Bereich oder Creutzfeldt-Jacob-Erkrankungen oder Apoplex oder Herzrhythmusstörungen oder Herzinfarkt oder Interstitium oder Embolie oder Thrombose oder Wund- und Knochenheilungsstörungen oder cerebrale Durchblutungsstörungen oder cerebrale Stoffwechselstörungen wie Morbus Alzheimer oder Prostatahypertrophien oder Hypertrophien und Metaplasien anderer Organe oder Entzündungen von Organen oder Körperstrukturen oder chronische Entzündungen wie Multiple Sklerose bei der Ausheilung oder Macula- Degeneration oder Verdauungsstörungen oder Faltenbildung der Haut oder endokrine Erkrankungen der Niere, der Leber oder Diabetes.

Als Beispiel sei eine erfolgreiche Tumorbehandlung eines Patienten genannt, dessen Harnblase durch einen 15 cm großen Tumor vollständig mit Tumorgewebe ausgefüllt war, sodass sie keinen Urin mehr aufnehmen konnte. Der Tumor war bereits in die Wand der Blase eingewachsen. Dem Patient musste zur Schmerztherapie eine Morphiumdosis von 400 mg pro Tag verabreicht werden.

Der Patient wurde über einen Zeitraum von 2 Monaten hinweg mehrmals in der Woche mit einer Impulsserie von 3000 Impulsen aus einem erfindungsgemäßen Impulswellengenerator mit einem Druckfeldgenerator und einem elektromagnetischen Feldgenerator ausgesetzt.

Nach dieser Behandlung konnte im Kernspin gezeigt werden, dass sich der Tumor wieder vollständig zurückgebildet hatte und die Biasenwand auf ihre ursprüngliche Wandstärke reduziert worden war. Auf die Gabe von Morphium konnte vollständig verzichtet werden. Im Kernspin ebenso wie in der Funktion war nachweisbar, dass sich die Harnblase wieder mit einem normalen Harnvolumen füllte. Nebenwirkungen während der Behandlung konnten nicht verzeichnet werden.

Das steht in überaus deutlichem Gegensatz zur bisher üblichen Behandlungsmethode der Chemotherapie, die massive Schäden an anderen, nicht betroffenen Organen und Körperregionen verursacht und dennoch nur einem Teil der Patienten ein Überleben ermöglicht.

Als Wirkungsmechanismus der Behandlung mit einem erfindungsgemäßen Impulswellengenerator konnten biochemische Untersuchungen zeigen, dass der anaerobe Stoffwechsel der Tumorzellen während der Behandlung wieder auf einen aeroben Stoffwechsel zurück geführt werden konnte, also auf ein Verhalten wie bei "normaler" Zellen. Es ist davon auszugehen, dass die Tumorzellen durch diese Veränderungen wieder für körpereigene "Reparaturvorgänge" angreifbar wurden.

Ein weiteres Anwendungsbeispiel sind 20 weibliche Patienten mit einem Mammacarzinom. Bei 18 Patienten waren die Knoten bereits größer als 3 cm.

Nach einer Behandlung mit dem erfindungsgemäßen Impulswellengenerator über 2 Monate hinweg mit wenigstens 3 Impulsserien pro Woche mit jeweils 3000 Impulsen hatten sich die Knoten vollständig zurückgebildet. Durch Nachkontrollen mit MRT zeigte sich, dass bei jeder Patientin auch in den folgenden 5 Jahren kein pathologischer Befund mehr nachweisbar war. Während der Behandlung wurden die Schmerzsymptome kontinuierlich reduziert und kehrten nach Abschluss der Behandlung weder als Druck- noch als Dauerschmerz zurück.

Ein weiteres Anwendungsgebiet eines erfindungsgemäßen Impulswellengenerators ist die indirekte Therapie eines inneren Organs über das jeweils zugehörige vegetative Nervensystem. Dabei betragen die zeitlichen Abstände zwischen zwei aufeinander folgenden Impulsen und Impulsserien eine bis zehn Minuten. Über die afferenten, hinteren Wurzeln der Spinalnerven eines Organs werden nicht nur afferente, vegetative Fasern aus den jeweils zugehörigen Organbezirken sondern auch von Hautsegmenten (Dermatomen) geführt. Zu jeder Erkrankung gehören deshalb überempfindliche, schmerzhafte Hautbezirke, auch als Head'sche Zonen bezeichnet. Anhand dieser Zonen kann das für die jeweilige Erkrankung maßgebliche Ganglion ermittelt und mit der impulsartigen Welle beaufschlagt werden. Erfolgreich behandelt wurden die Ganglien coeliacum inferior, cervicale inferior, cervicale superior, sacrale und pelvinum.

Alternativ kann auch eine Head'sche Zone selbst als neural sensibler Bereich mit einer erfindungsgemäßen Impulswelle beaufschlagt werden. Auf beide Arten wird das zugehörige Organ therapeutisch beeinflusst.

Als ein Nachweis der Wirkung ist im gesamten Organismus eine Änderung des Spiegels an Neurotransmittern und biogenen Aminen zu beobachten. Siehe dazu W. Kloepfer und Weth, G. et al. "Atlastherapy and Neurotransmitters in Patients affected by Multiple Sclerosis", manuelle Medizin, 27. Jahrgang, Heft 4, Seite 82, August 1989, Springer Verlag.

Eine weitere, effiziente Anwendung eines erfindungsgemäßen Impulsgenerators ist die Behandlung von Schmerzen, insbesondere in Folge von Tumoren, rheumatoiden Erkrankungen und deren Therapie oder Verspannungen oder Durchblutungsstörungen z.B. der Beine als Folge von Stoffwechselerkrankungen oder Herzinfarkt und Schlaganfall oder vasospastischen Erkrankungen oder internistischen Erkrankungen wie Morbus Wipple, Angina Pectoris, Magenschmerzen oder funktionelle Oberbauchbeschwerden oder neurologischen und psychiatrischen Erkrankungen wie Neuralgien, Multipler Sklerose oder dem neurovegetativem Syndrom.

Ein erfindungsgemäßer Impulswellengenerator kann durch die Auswahl eines passenden Fokussierungspunktes und die entsprechende Einstellung der Intensität und des Zeitpunktes jedes Feldimpulses sogar auf die Modulation des Stoffwechsels der Zelle eingestellt werden und zwar auf den mitochondrialen, zellulären oder extrazellulären Stoffwechsel oder die Zellmatrix.

Bei der Anwendung des erfindungsgemäßen Impulsgenerators hat es sich als besonders wirkungsvoll herausgestellt, wenn jeder einzelne Impuls eine Dauer von maximal 0,1 Sekunden hat. Die beste Wirkung wurde mit einem einzelnen Impuls erzielt, der eine Gesamtdauer von 70-100 Millisekunden aufwies.

Bei vielen Erkrankungen, Störungen und Schmerzen erwies sich der folgende Behandlungsplan als am wirkungsvollsten: Bis zu jeweils 10.000 einzelne Impulse der Feldgeneratoren wirken in einem Abstand von maximal 0,5 Sekunden auf den zu behandelnden Fokussierungspunkt des Körpers ein. Diese Impulsserie wurde im Abstand von einem Tag bis zu einer Woche über einen Zeitraum von bis zu 3 Monaten wiederholt.

Als Variante wurde bei einigen Anwendungen jeder einzelne Impuls zu einer Impulsserie von bis zu 100 Millisekunden Dauer erweitert.

Durch einen erfindungsgemäßen Impulswellengenerator kann - wie erwähnt - die Matrix einer nicht gesunden Zelle so moduliert werden, dass sich ihr extrazellulärer Stoffwechsel dem einer gesunden Zelle wieder annähert, was durch eine verbesserte Durchblutung feststellbar ist, die durch eine Erhöhung des Dopamingehaltes nachweisbar ist. Dopamin ist einer der wichtigsten Neurotransmitter insbesondere im Gehirnstoffwechsel.

Bei Zellen, die durch einen Schlagfall, einen Herzinfarkt oder eine Tumorerkrankung geschädigt worden sind, konnte gemessen werden, dass der Dopaminspiegel vor der Behandlung mit einem erfindungsgemäßen Impulswellengenerator deutlich niedriger war als nach der Behandlung.

Ein weiteres Indiz für die positive Wirkung des erfindungsgemäßen Impulswellengenerators ist die dramatische Reduzierung des täglichen Erbrechens - wie es für Karzinompatienten, die sich einer Chemotherapie oder einer Strahlentherapie unterziehen, typisch ist. - vor und nach einer Impulswellenbehandlung.

Im Folgenden sollen weitere Einzelheiten und Merkmale der Erfindung anhand eines Beispiels näher erläutert werden. Dieses soll die Erfindung jedoch nicht einschränken, sondern nur erläutern.

Es zeigt in schematischer Darstellung:
- Figur 1: Längsschnitt durch einen erfindungsgemäßen ImpulsWellengenerator
- Figur 2: Prozentualer Anstieg von Dopamin durch die Impulswellenbehandlung betroffener Zellen bei Schlaganfällen
- Figur 3: Reduzierung des täglichen Erbrechens von Karzinompatienten durch die Impulswellenbehandlung

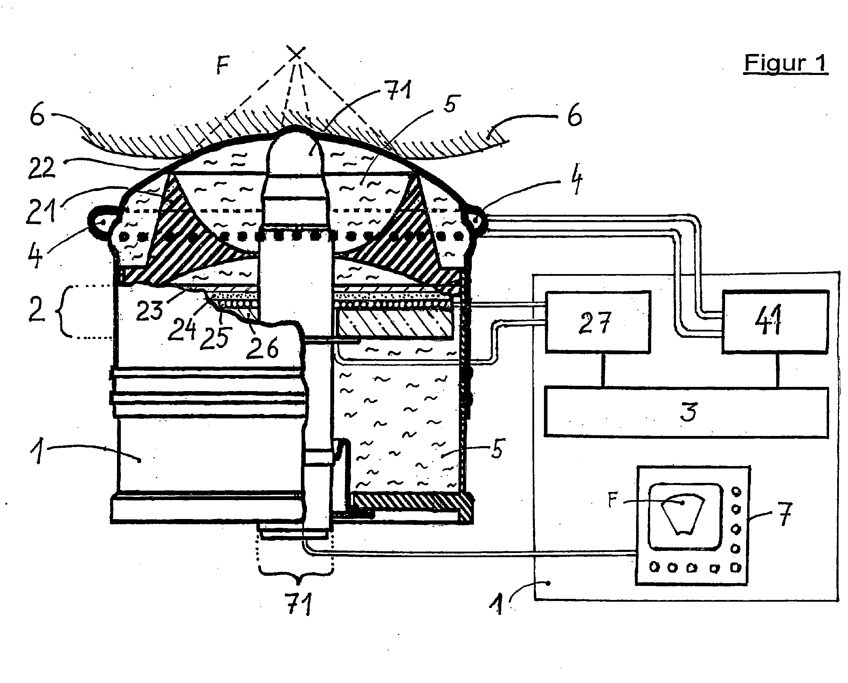

In Figur 1 ist das Gehäuse 1 in zwei Teilen dargestellt: In der rechten Hälfte ist das Gehäuse 1 nur als Blockschaltbild mit darin enthaltenen vier elektrischen Funktionsbaugruppen dargestellt, links ist ein weiterer Teil des Gehäuses 1 als Realitätsnahes Schnittbild gezeigt, in dem diejenigen Baugruppen angeordnet sind, die das Druckfeld des Druckfeldgenerators 2 erzeugen, fokussieren und übertragen sowie ein elektromagnetisches Feld erzeugen und abstrahlen.

In der linken, realitätsnahen Hälfte von Figur 1 ist der etwa topfförmige Teil eines - in diesem Ausführungsbeispiel rotationssymmetrischen - Gehäuses 1 zeichnerisch teilweise aufgebrochen dargestellt, sodass der Blick auf den darin enthaltenen Druckfeldgenerator 2 und den darauf aufgelegten Feldgenerator 4, hier zur Erzeugung eines elektromagnetischen Feldes, frei wird. Dieser Teil des Gehäuses 1 ist mit der Übertragungsflüssigkeit 5 befüllt, die die Druckimpulse vom Druckfeldgenerator 2 auf die Druckimpulsfokussierung 21-auch akustische Linse genannt - überträgt. Dort werden sie auf den Fokussierungspunkt F fokussiert und vom Druckimpulskoppler 22 auf den Körper 6 übertragen.

In Figur 1 ist gut zu erkennen, dass der Querschnitt der Druckimpulsfokussierung dem Querschnitt eines Brillenglases ähnelt. Deshalb wird diese Einheit auch "akustische Linse" genannt. Das Funktionsprinzip ähnelt auch dem eines optischen Glases. Entsprechend zu dem sogenannten "Brechungsindex" des Brillenglases, der sich von dem der Umgebung deutlich unterscheidet, ist auch hier die Schallgeschwindigkeit innerhalb des festen Materials der akustischen Linse deutlich anders als die Schallgeschwindigkeit in der - die Linse umgebenden - Übertragungsflüssigkeit 5. Dadurch ergibt sich, dass die Druckimpulse mit einem anderen Winkel zur Oberfläche eintreten, als sie austreten.

Die von der akustischen Linse gebündelten Druckimpulse werden an den Druckimpulskoppler 22 weitergeleitet, auch "Akustikkoppler" genannt. In Figur 1 ist gut zu sehen, dass der Druckimpulskoppler 22 aus einem - flexiblen - Beutel besteht, der mit der Übertragungsflüssigkeit 5 befüllt ist. Dadurch werden die von der Druckimpulsfokussierung 21 abgegebenen Schallwellen fast vollständig auf den Fokussierungspunkt F im Körper 6 des Patienten übertragen. In Figur 1 ist der Druckimpulskoppler 22 im Querschnitt zu sehen; seine flexible Außenschicht ist durch eine breite schwarze Linie dargestellt.

Mit gestrichelten Linien ist der Verlauf der Druckimpulswellen in den Fokussierungspunkt F gekennzeichnet. In diesen Fokussierungspunkt F muss die *von* einer Krankheit oder Störung befallene Region des Körpers justiert werden. Die in Figur 1 teilschraffierte Randzone des Körpers 6 könnte also z. B. eine Brust darstellen, die von einem Tumor befallen ist, der am Ort des Fokussierungspunktes F wuchert.

In Figur 1 ist als Ausführungsbeispiel für den Druckfeldgenerator 2 eine elektromagnetische Druckerzeugung dargestellt: Im Schnitt ist die Membrane 23 zu erkennen, die elektrisch leitende Bestandteile enthält. Sie ist durch die Isolierfolie 24 von der Flachspule 25 getrennt, die auf dem Spulenträger 26 aufliegt. Die Flachspule 25 besteht in diesem Fall aus einem spiralförmig auf dem Spulenträger 26 verlegten, elektrischen Leiter, der im Querschnitt als aneinandergereihte Kreise zu erkennen ist. Diese Spule ist über zwei Leitungen an den Hochspannungsimpulsgenerator 27 angeschlossen, der nur schematisch dargestellt ist.

Auf dem realitätsnah dargestellten Gehäuse 1 erkennt man den weiteren Feldgenerator 4, in diesem Ausführungsbeispiel ein ebenso wie der Druckfeldgenerator 2 elektromagnetischer Feldgenerator. Er besteht aus einer ringförmigen Spule, die um den oberen Rand des hier ebenfalls etwa kreisförmigen Gehäuses 1 montiert ist. Links und rechts am Gehäuse 1 ist die Spule des Feldgenerators 4 im Schnitt zu erkennen. Mit großen Punkten und einer gestrichelten Linie ist der Verlauf der Spule über das Gehäuse 1 hinweg gekennzeichnet. Diese Spule ist über zwei elektrische Leiter an die in Figur 1 nur schematisch dargestellte Versorgungseinheit 41 angeschlossen. Diese Versorgungseinheit 41 versorgt die Spule mit Stromimpulsen von sehr hoher Stromstärke und sehr hoher Spannung. In Figur 1 ist gut nachvollziehbar, dass sich daraufhin in der Spule ein elektromagnetisches Feld ausbildet, dass sich zum Körper 6 hin ungehindert ausbreitet und den Fokussierungspunkt F durchdringt. Zur anderen Seite hin wird es durch das metallisch leitende Gehäuse 1 reflektiert und ebenfalls in den Körper 6 geleitet.

In Figur 1 sind also zwei Feldgeneratoren eingetragen: Zum einen der mechanische Druckfeldgenerator 2 und zum anderen der weitere Feldgenerator 4, hier in der Ausführungsform als elektromagnetischer Feldgenerator dargestellt. Die Versorgungseinheiten beider Feldgeneratoren sind zum einen der Hochspannungsimpulsgenerator 27 und zum anderen der Stromimpulsgenerator für die Spule, hier als Versorgungseinheit 41 bezeichnet. Beide werden von der Steuerung 3 gesteuert.

In Figur 1 ist nicht eingezeichnet, dass die Steuerung dafür sorgt, dass die Impulse oder Impulsfolgen eine Länge von maximal 100 Millisekunden haben und je nach Art der zu behandelnden Störung oder Krankheit nach typischer Weise 500 Millisekunden Pause den nächsten Impuls auslösen und - je nach zu behandelnder Krankheit oder Störung - bis zu 10.000 Impulse nacheinander aktivieren.

In Figur 1 ist als weitere, optionale Ausstattung eines erfindungsgemäßen Impulswellengenerators ein Ultraschalldiagnosegerät 7 im schematisch dargestellten Teil des Gehäuses 1 eingezeichnet. Der zugehörige Schallkopf 71 ist in der Längsachse des Gehäuses 1 angeordnet und befindet sich damit auch in der Längsachse des Druckfeldgenerators 2 und des weiteren Feldgenerators 4.

Die Spitze des Schallkopfes 71 ragt geringfügig über die flexible Außenhaut des Druckimpulskopplers 22 heraus. Zusätzlich ist der Schallkopf 71 noch gegen das Gehäuse 1 um seine Längsachse verschwenkbar. Dadurch wird auf dem Monitor des Ultraschalldiagnosegerätes 7 der Bereich sichtbar, in dem die beiden Feldgeneratoren 2 und 4 tätig sind, sodass kontrolliert werden kann, ob der Fokussierungspunkt F tatsächlich den erkrankten Bereich des Körpers, z. B. einen Tumor, trifft. Zur Arbeitserleichterung ist deshalb der Fokussierungspunkt F auf dem Bildschirm des Ultraschalldiagnosegerätes 7 markiert.

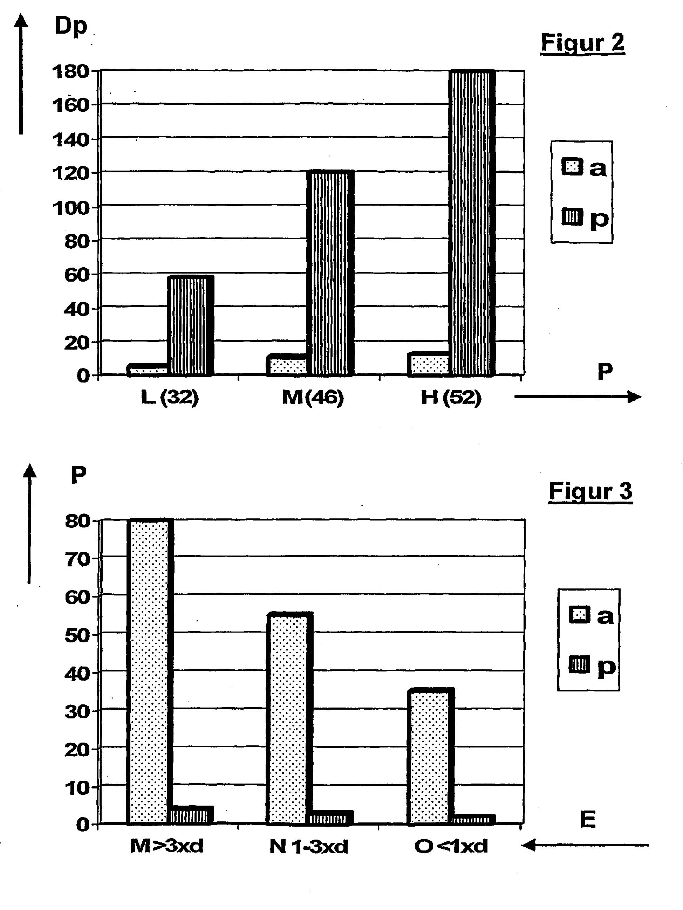

In Figur 2 ist der gemessene prozentuale Anstieg von Dopamin durch die Impulswellenbehandlung geschädigter Zellen bei Schlaganfall-Patienten P als Diagramm gezeigt.

Eine Menge von 130 Patienten P wurde abhängig von ihrem Dopaminspiegel Dp nach der Impulswellenbehandlung in drei Gruppen aufgeteilt und zwar in eine Gruppe H, bei der sich der Dopaminspiegel Dp durch die Behandlung auf etwa das Doppelte des Normalwertes von 100% erhöhte. H steht für "Hoch". Dieser Gruppe wurden 52 Patienten P zugewiesen. Der exakte Mittelwert des Dopaminspiegel Dp dieser 52 Patienten P in der Gruppe H stieg durch die Behandlung auf 180 % des Normalwertes von 100%.

Der Dopaminspiegel Dp einer zweiten Gruppe L von Patienten P hatte auch nach der Behandlung nur etwa die Hälfte des Normalwertes von 100 %. L steht hier für "Low" oder "Niedrig." Dieser Gruppe wurden 32 Patienten P zugewiesen. Der exakte Mittelwert des Dopaminspiegels Dp dieser Patienten P stieg durch die Behandlung auf 58 % des Normalwertes von 100%.

Bei den 46 übrigen Patienten P stieg der Dopaminspiegel Dp nach der Behandlung etwa auf den normalen Wert an. Diese Gruppe wird mit M für "Mittel" bezeichnet. Der exakte Mittelwert des Dopaminspiegel Dp dieser Patienten P stieg durch die Behandlung auf 120 % des Normalwertes von 100% an.

In der ersten Gruppe L stieg der Dopaminspiegel von mittleren 5,5% auf 58 % des Normalwertes, also auf das 10,5-fache des Ausgangswertes. In der zweiten Gruppe M erhöhte sich der Dopaminspiegel von mittleren 11 % auf 120 %,also auf das 10,9-fache des Ausgangspegels. Und in der Gruppe H war der relative Anstieg von mittleren 13% auf 180 % mit dem 13,8-fachen am höchsten.
In Figur 2 ist auf der waagerechten Achse die jeweilige Anzahl P von Patienten in den drei Gruppen L, M und H wiedergegeben. Auf der senkrechten Achse ist für jede Gruppe der mittlere Dopaminspiegel Dp im Blut in Bezug auf den jeweiligen Normalwert des Dopaminspiegels Dp als Säule eingezeichnet, und zwar mit "a" vor und mit "p" nach der Behandlung mit einem erfindungsgemäßen Impulswellengenerator.

Figur 2 zeigt, dass bei der Krebsbehandlung mit einem erfindungsgemäßen Impulswellengenerator der Dopaminspiegel Dp auf wenigstens das 10-fache des Ausgangswertes erhöht werden kann, was einen dramatischen Schritt zurück zu einem normalen Stoffwechsel der zuvor kranken Zellen anzeigt.

In Figur 3 wird die Reduzierung des täglichen Erbrechens E von Karzinompatienten P durch die Impulswellenbehandlung dargestellt. Auf der waagerechten Achse ist die tägliche Häufigkeit des Erbrechens E eingetragen: Links die größte Häufigkeit, rechts die geringste Häufigkeit. Auf der senkrechten Achse die Anzahl der Patienten P, die unter einer solchen Häufigkeit des täglichen Erbrechens leiden.

Zur Untersuchung wurde eine Menge von 170 Patienten P in drei Gruppen aufgeteilt. Der Gruppe M wurden alle 80 Patienten zugewiesen, die sich öfter als 3-mal am Tag übergeben mussten. Die 55 Patienten der Gruppe N erbrachen sich 1- bis 3-mal am Tag und die 35 Patienten der Gruppe nicht an jedem Tag, also weniger als 1-mal täglich.

In Figur 3 ist mit "a" die Anzahl der Patienten P vor und mit "p" die Anzahl nach der Behandlung mit einem erfindungsgemäßen Impulswellengenerator gekennzeichnet.

In der am stärksten betroffenen Gruppe M senkt die Behandlung die Anzahl der Patienten P, die noch immer so oft erbrechen müssen, von 80 auf 4, also um 95%. In der mittleren Gruppe M sinkt der Anteil der weiterhin so stark betroffenen Patienten von 55 auf 3, also um 94% und in der am wenigsten betroffenen Gruppe O von 35 auf 2 Patienten P, also ebenfalls eine Reduzierung um 94 %.

Damit zeigt die Figur 3 eindrucksvoll, dass die Behandlung mit einem erfindungsgemäßen Impulswellengenerator auch die Nebenwirkungen einer Karzinomerkrankung spürbar vermindern kann.

### Bezugszeichenliste

### Ansprüche und Figur 1

- F: Fokussierungspunkt des Druckfeldgenerators 2 im oder am Körper 6
- 1: Gehäuse zur Aufnahme der Feldgeneratoren 2 und 4
- 2: Druckfeldgenerator, im Gehäuse 1
- 21: Druckimpulsfokussierung zwischen Druckfeldgenerator 2 und Druckimpulskoppler 22
- 22: Druckimpulskoppler, zwischen Druckimpulsfokussierung 21 und Körper 9
- 23: Membran des Druckfeldgenerators 2
- 24: Isolierfolie, zwischen Membran 23 und Flachspule 25
- 25: Flachspule, zur Erzeugung des Druckimpulses durch Abstoßung der Membran 23
- 26: Spulenträger, trägt Flachspule 25
- 27: Hochspannungsimpulsgenerator zur Aktivierung der Flachspule 25
- 28: Übertragungsflüssigkeit, zwischen Membran 23 und Druckimpulsfokussierung 21 und zwischen Druckimpulsfokussierung 21 und Druckimpulskoppler 22
- 3: Steuerung der Feldgeneratoren 2 und 4
- 4: weiterer Feldgenerator, erzeugt ein Feld im Fokussierungspunkt F
- 41: Versorgungseinheit des Feldgenerators 4
- 5: Übertragungsflüssigkeit, zwischen Membran 23 und Druckimpulsfokussierung 21 und zwischen Druckimpulsfokussierung 21 und Druckimpulskoppler 22
- 6: Körper mit Fokussierungspunkt F, wo die Felder der Feldgeneratoren 2 und 4 wirken
- 7: Ultraschalidiagnosegerät
- 71: Schallkopf des Ultraschalldiagnosegeräts 7, integriert in Druck feldgenerator 2

### Figur 2 und 3

- a: vor der Impulswellenbehandlung
- p: nach der Impulswellenbehandlung

### Figur 2

- Dp: Dopaminspiegel in % des Normalwertes
- P: Anzahl der Patienten
- L: Patientengruppe mit relativ niedrigem Dopaminspiegel
- M: Patientengruppe mit mittlerem Dopaminspiegel
- H: Patientengruppe mit hohem Dopaminspiegel

### Figur 3

- P: Anzahl der Patienten
- E: Erbrechenshäufigkeit
- M >3xd: öfter als 3-mal tägliches Erbrechen
- N 1-3xd: Erbrechen 1-3-mal täglich
- O <1xd: weniger als 1-mal Erbrechen pro Tag

## Patentansprüche

1. Impulswellengenerator, bestehend aus einem Gehäuse (1) mit
- einem Druckfeldgenerator (2) von dem einzeln ansteuerbare Druckimpulse abstrahlbar sind und
- einer Druckimpulsfokussierung (21), mittels derer die Druckimpulse in einem Fokussierungspunkt F in oder an einem Körper 6 eines Lebewesens fokussierbar sind und
- einem Druckimpulskoppler (22) von dem die Druckimpulse auf den Körper (6) übertragbar sind und
- einer Steuerung (3) für die Intensität und den Zeitpunkt jedes Druckimpulses, wobei
wenigstens ein weiterer Feldgenerator (4) integriert ist, durch den
- einzeln ansteuerbare Feldimpulse im Fokussierungspunkt F erzeugbar sind und
- die Intensität und der Zeitpunkt jedes Feldimpulses steuerbar ist.
**dadurch gekennzeichnet, dass**
als Feldgenerator (4) zur Erzeugung eines Magnetfeldes eine Spule dient, die durch die Entladung eines auf bis zu 50 kV aufgeladenen Kondensators
- über eine Plasmakammer oder
- über eine Funkenstrecke
von einem sehr hohen Stromimpuls von bis zu 3 kA durchflossen wird,
woraus sich ein elektromagnetischer Impuls ergibt,
- dessen Energieinhalt kurzzeitig sehr hoch ist und
- der durch Induktion das effektive Transmembranpotential einer Tumorzelle kurzzeitig auf einen normalen Wert anhebt.

2. Impulswellengenerator nach Anspruch 1, **dadurch gekennzeichnet, dass** das Transmembranpotential einer Tumorzelle von 15 bis 20 mV kurzzeitig auf das Transmembranpotential einer gesunden Zelle von etwa 80 bis 100 mV anhebbar ist.

3. Impulswellengenerator nach Anspruch 1, **dadurch gekennzeichnet, dass** das Transmembranpotential einer durch chronischen Schmerz stark gereizten Zelle von etwa 60 bis 80 mV kurzzeitig auf das Transmembranpotential einer gesunden Zelle von etwa 80 bis 100 mV anhebbar ist.

4. Impulswellengenerator nach einem der vorhergehenden Ansprüche, **dadurch gekennzeichnet, dass**
von jedem der weiteren Feldgeneratoren (4)
- ein magnetisches oder
- ein elektrisches oder
- ein elektromagnetisches
Feld erzeugbar ist.

5. Impulswellengenerator nach einem der vorhergehenden Ansprüche, **dadurch gekennzeichnet, dass** von wenigstens einem der Feldgeneratoren (2) oder (4) ein Wechselfeld erzeugbar ist, dessen
- Amplitude und
- Frequenz und
- Einschaltdauer und
- Einschaltzeitpunkt
steuerbar ist.

6. Impulswellengenerator nach dem vorhergehenden Anspruch 3,
**dadurch gekennzeichnet, dass** die Amplitude und/oder die Frequenz über die Einschaltdauer hinweg
- ansteigt und/oder
- abfällt und/oder
- oszilliert.

7. Impulswellengenerator nach einem der vorhergehenden Ansprüche, **dadurch gekennzeichnet, dass** in der Längsachse des Druckfeldgenerators (2) ein Schallkopf (71) eines Ultraschall-Diagnose-Gerätes (7) angeordnet ist,
- der durch die Druckimpulsfokussierung (21) und den Druckimpulskoppler (22) hindurch etwas über die Außenfläche des Druckimpulskopplers (22) hinaus ragt und
- etwas in den Körper (6) hinein drückbar ist und
- die Messebene des Schallkopfes (71) durch den Fokussierungspunkt F verläuft.

8. Impulswellengenerator nach einem der vorhergehenden Ansprüche,
**dadurch gekennzeichnet, dass** der Druckfeldgenerator (2) eine dauernd elastische Membran ist,
- die zumindest an mehreren Punkten ihres Umfanges eingespannt ist und
- in ihrem Zentrum oder nahe dazu durch einen mechanischen Impuls auslenkbar ist, der
- etwa senkrecht zur Fläche der Membran und
- auf den Fokussierungspunkt F
gerichtet ist.

9. Impulswellengenerator nach dem vorhergehenden Anspruch 8,
**dadurch gekennzeichnet, dass** der mechanische Impuls durch
- einen Elektromagneten oder
- einen Piezokristall oder
- eine vorgespannte Feder oder
- einen pneumatischen oder hydraulischen Druckspeicher mit einem Zylinder oder
- das Entzünden eines explosiven Treibmittels oder
- das Verschwenken eines Nockens
erzeugt wird.

10. Impulswellengenerator nach einem der vorhergehenden Ansprüche,
**dadurch gekennzeichnet, dass** der Druckfeldgenerator (2) eine in Richtung des Fokussierungspunktes F bewegbare Masse ist, die durch einen mechanischen Impuls in Richtung des Fokussierungspunktes F ausgelenkt wird.

11. Impulswellengenerator nach dem vorhergehenden Anspruch 10.
**dadurch gekennzeichnet, dass**
von der bewegbaren Masse
- eine kinetische Energie von wenigstens 5 Millijoule abgebbar ist und
- eine Geschwindigkeit von wenigstens 3 m/s erreichbar ist und
die Masse des Festkörpers weniger als 0,1g betragen kann.

12. Impulswellengenerator nach den vorhergehenden Ansprüchen 10 oder 11, **dadurch gekennzeichnet, dass** als Druckimpulsfokussierung eine Mulde oder Vertiefung auf der zum Fokussierungspunkt F weisenden Seite der bewegten Masse dient.

13. Impulswellengenerator nach dem vorhergehenden Anspruch 12,
**dadurch gekennzeichnet, dass** aus der Mulde oder Vertiefung ein Luftkanal heraus bis an eine vom Körper (6) abgewandte Seite der bewegten Masse führt.

14. Impulswellengenerator nach einem der vorhergehenden Ansprüche, **dadurch gekennzeichnet, dass** der Druckimpulskoppler (22) aus einem dauerhaft flexiblen und mit einer Übertragungsflüssigkeit (5) befülltem Beutel besteht, der
- mit einer Seite an den Körper (6) und
- mit der gegenüberliegenden Seite an die Druckimpulsfokussierung (21) angrenzt.

15. Impulswellengenerator nach einem der vorhergehenden Ansprüche, **dadurch gekennzeichnet, dass**
die Druckimpulsfokussierung (21) eine akustische Linse ist, die aus einem harten und schwingungsbeständigem Material in Form einer Scheibe besteht,
- deren Rand im Gehäuse (1) so eingespannt ist, dass sie quer zur Richtung des Druckimpulses ausgerichtet ist und
- auf wenigstens einer Seite eine konkave Vertiefung aufweist, die rotationssymmetrisch zur Richtung des Druckimpulses geformt ist und
- auf beiden Seiten jeweils vollflächigen Kontakt mit einer Übertragungsflüssigkeit (5) hat,
- die auf der einen Seite den Druckimpulskoppler (22) füllt und
- auf der zweiten Seite an den Druckfeldgenerator (2) angrenzt und
- die Oberflächen der Scheibe so geformt sind, dass sie jeden Teil
eines Druckimpulses aus dem Druckfeldgenerator (2) im Fokussierungspunkt F bündeln.

16. Impulswellengenerator nach einem der vorhergehenden Ansprüche, **dadurch gekennzeichnet, dass** der Druckfeldgenerator (2) aus einer ebenen Membran (23) besteht,
- die elektrisch leitfähiges Material, wie z.B. Kupfer oder Aluminium enthält und
- mit der zum Körper (6) weisenden Seite an die im Gehäuse 1 befindliche Übertragungsflüssigkeit (5) angrenzt und
- mit der andere Seite auf einer Isolierfolie (24) aufliegt,
- die auf einer spiralförmig gewickelte Flachspule (25) aufliegt,
- welche von einem Spulenträger (26) gehalten wird und
- von einem Hochspannungsimpulsgenerator (27) mit Hochspannungsimpulsen beaufschlagbar ist.

17. Impulswellengenerator nach einem der vorhergehenden Ansprüche, **dadurch gekennzeichnet, dass** der Druckfeldgenerator (2) Drücke von wenigstens 0,6 bis zu 6 dyn/mm² erzeugt.

18. Impulswellengenerator nach Anspruch 16, **dadurch gekennzeichnet, dass**
die Stromstärke des Hochspannungsimpulses größer als 1 kA ist und die Spannung größer als 1kV ist.

19. Impulswellengenerator nach einem der vorhergehenden Ansprüche, **dadurch gekennzeichnet, dass** ein Apparat zur Infusion flüssiger Wirkstoffe integriert ist.

20. Impulswellengenerator nach einem der vorhergehenden Ansprüche, **dadurch gekennzeichnet, dass** er implantierbar ist.

21. Impulswellengenerator nach einem der vorhergehenden Ansprüche, **dadurch gekennzeichnet, dass** in der Steuerung (3) einstellbar ist, dass jeder einzelne Impuls der Feldgeneratoren (2) und/oder (4) eine Dauer von max 0,1 sec hat.

22. Impulswellengenerator nach einem der vorhergehenden Ansprüche, **dadurch gekennzeichnet, dass** in der Steuerung (3) einstellbar ist, dass
- bis zu 10.000 einzelne Impulse der Feldgeneratoren (2) und/oder (4) im Abstand von maximal 0,5 sec auf den zu behandelnden Fokussierungspunkt F des Körpers einwirken und
- diese Impulsserien im Abstand von einem Tag bis zu einer Woche über einen Zeitraum von bis zu 3 Monaten wiederholt werden.

23. Impulswellengenerator nach einem der vorhergehenden Ansprüche, **dadurch gekennzeichnet, dass** in der Steuerung einstellbar ist, dass jeder einzelne Impuls zu einer Impulsserie von bis zu 0,1 sec Dauer erweitert wird.

24. Impulswellengenerator nach einem der vorhergehenden Ansprüche, **dadurch gekennzeichnet, dass** der Ort des Fokussierungspunktes F und/oder die Intensität und/oder der Zeitpunkt jedes Feldimpulses auf die Behandlung von Tumoren und Thromben und anderen, in einem begrenzten Raum lokalisierten Beschwerden oder Schmerzempfindungen im oder am Körper (6) von Lebewesen einstellbar ist.

25. Impulswellengenerator nach einem der vorhergehenden Ansprüche, **dadurch gekennzeichnet, dass**
- der Ort des Fokussierungspunktes F und/oder
- die Intensität jedes Feldimpulses und/oder
- der Zeitpunkt jedes Feldimpulses
auf die Behandlung von
- Tumoren in der Brust oder in der Bauchspeicheldrüse oder im Gehirn oder in der Prostata oder in einem anderen Organ oder Bereich oder
- Creutzfeldt-Jacob-Erkrankung oder
- Apoplex oder
- Herzrhythmusstörungen oder
- Herzinfarkt oder
- Interstitium oder
- Embolie oder
- Thrombose oder
- Wund- und Knochenheilungsstörungen oder
- cerebralen Durchblutungsstörungen oder
- cerebralen Stoffwechselstörungen wie Morbus Alzheimer oder
- Prostatahypertrophien oder
- Hypertrophien und Metaplasien anderer Organe oder
- Entzündungen von Organen oder Körperstrukturen oder
- Ausheilung chronischer Entzündungen wie Multipler Sklerose oder
- Macula- Degeneration oder
- Verdauungsstörungen oder
- Faltenbildung der Haut oder
- endokrinen Erkrankungen der Niere oder der Leber oder
- Diabetes
einstellbar ist.

26. Impulswellengenerator nach einem der vorhergehenden Ansprüche, **dadurch gekennzeichnet, dass**
- der Ort des Fokussierungspunktes F und/oder
- die Intensität jedes Feldimpulses und/oder
- der Zeitpunkt jedes Feldimpulses
zur indirekten Therapie eines inneren Organs auf
- das jeweils zugehörige Ganglion coeliacum inferior oder cervicale inferior oder cervicale superior oder sacrale oder pelvinum oder
- auf den jeweils zugehörigen Head'schen Hautbezirk einstellbar ist.

27. Impulswellengenerator nach einem der vorhergehenden Ansprüche, **dadurch gekennzeichnet, dass**
- der Ort des Fokussierungspunktes F und/oder
- die Intensität jedes Feldimpulses und/oder
- der Zeitpunkt jedes Feldimpulses
auf die Therapie von Schmerzen infolge von
- Tumoren, rheumatoiden Erkrankungen und deren Therapie oder
- Verspannungen oder
- Durchblutungsstörungen z.B. der Beine als Folge von Stoffwechselerkrankungen oder
- Herzinfarkt und Schlaganfall oder
- vasospastischen Erkrankungen oder
- internistischen Erkrankungen wie Morbus Wipple, Angina Pectoris, Magenschmerzen oder funktionellen Oberbauchbeschwerden oder
- neurologischen und psychiatrischen Erkrankungen wie Neuralgien,
Multipler Sklerose, neurovegetativem Syndrom einstellbar ist.

28. Impulswellengenerator nach einem der vorhergehenden Ansprüche, **dadurch gekennzeichnet, dass**
- der Ort des Fokussierungspunktes F und/oder
- die Intensität jedes Feldimpulses und/oder
- der Zeitpunkt jedes Feldimpulses auf die Modulation des
- mitochondrialen oder
- zellulären oder
- extrazellulären
Stoffwechsels oder der Zellmatrix einstellbar ist.

## Claims

1. Pulse wave generator, comprising a housing (1) having a pressure field generator (2) by which individually controllable pressure pulses can be emitted, and having a pressure pulse focusing unit (21) by way of which the pressure pulses can be focused in a focusing point (F) in or on a body (6) of a living being, and having a pressure pulse coupler (22) by which the pressure pulses can be transmitted to the body (6) and having a controller (3) for the intensity and the point of time of each pressure pulse, wherein at least one further field generator (4) is integrated, by which individually controllable field pulses can be generated in the focusing point (F) and the intensity and point of time of each pulse can be controlled,
**characterized in that**
as a field generator (4) for generating a magnetic field a coil is used, which by the discharge of a charged up to 50 kV capacitor via a plasma chamber or via a spark gap is flowed through by a very high current pulse of up to 3 kA, resulting in an electromagnetic pulse, whose energy content is temporarily very high and who lifts by induction the effective transmembrane potential of a tumor cell or another, strongly irritated cell temporarily briefly to a normal level.

2. Pulse wave generator according to claim 1,
**characterized in that**
the transmembrane potential of a tumor cell of 15 to 20 mV is briefly lifted to the transmembrane potential of a healthy cell of about 80 to 100 mV.

3. Pulse wave generator according to claim 1,
**characterized in that**
the transmembrane potential of about 60 to 80 mV of a cell, being highly irritated by chronic pain, is briefly lifted to the transmembrane potential of a healthy cell from about 80 to 100 mV.

4. Pulse wave generator according to one of the preceding claims, **characterized in that**
of each of the further field generators (4) a magnetic or an electric or an electromagnetic field can be produced.

5. Pulse wave generator according to one of the preceding claims, **characterized in that**
at least from one of the field generators (2) or (4) an alternating field can be generated, whose amplitude and frequency and on-time and turn-on instant can be controlled.

6. Pulse wave generator according to claim 3,
**characterized in that**
the amplitude and/or frequency during the switch-on time increases and/or decreases and/or oscillates.

7. Pulse wave generator according to one of the preceding claims, **characterized in that**
in the longitudinal axis of the pressure field generator (2) a transducer (71) of an ultrasonic diagnosis device (7) is disposed, which projects through the pressure pulse focusing unit (21) and through the pressure pulse coupler (22) beyond the outer surface of the pressure pulse coupler (22) and which can be pressed a bit into the body (6) and the measurement plane of the transducer (71) runs through the focusing point (F).

8. Pulse wave generator according to one of the preceding claims, **characterized in that**
the pressure field generator 2 is a permanently elastic membrane, which is clamped at least at several points of its periphery and can be deflected in its centre or close thereto by a mechanical pulse, which is directed about perpendicular to the surface of the membrane and the focusing point F.

9. Pulse wave generator according to claim 8,
**characterized in that**
the mechanical pulse is generated by an electromagnet or a piezoelectric crystal or a preloaded spring or a pneumatic or hydraulic pressure accumulator with a cylinder or the ignition of an explosive propellant or the pivoting of a cam.

10. Pulse wave generator according to one of the preceding claims, **characterized in that**
the pressure field generator (2) is a mass, which is movable in the direction of the focusing point (F) and which is deflected by a mechanical impulse in the direction of the focusing point (F).

11. Pulse wave generator according to the preceding claim 10, **characterized in that**
from the movable mass a kinetic energy of at least 5 millijoules can be emitted and a speed of at least 3 m/s can be reached and the mass of the solid body may be less than 0.1 g

12. Pulse wave generator according to the preceding claims 10 or 11, **characterized in that**
a trough or recess on the moved mass on the side facing to the focusing point (F) serves as a pressure pulse focusing unit (21).

13. Pulse wave generator according to the preceding claim 12, **characterized in that**
an air duct leads out of the trough or out of the recess to the moving mass to a side facing away from the body 6.

14. Pulse wave generator according to one of the preceding claims, **characterized in that**
the pressure pulse coupler (22) consists of a bag, which is permanently flexible and inflated with a transfer fluid (5) and adjoins with one side to the body (6) and with the opposite side to the pressure pulse focusing unit (21).

15. Pulse wave generator according to one of the preceding claims, **characterized in that**
the pressure pulse focusing unit (21) is an acoustic lens comprising a hard and vibrations enduring material in the form of a disc, whose edge is clamped in the housing (1) so that it is oriented transverse to the direction of the pressure pulse and which has at least on one side a concave recess, that is shaped rotationally symmetrical into the direction of the pressure pulse and which has on both sides respectively full-area contact with a transfer fluid (5), which fills on the first side the pressure pulse coupler (22) and adjoins on the second side to the pressure field generator (2) and the surfaces of the disc are formed so that they focus every part of a pressure pulse from the pressure field generator (2) in the focusing point F.

16. Pulse wave generator according to one of the preceding claims,**characterized in that**
the pressure field generator (2) consists of a plane membrane (23), which contains electrically conductive material, such as copper or aluminium and which adjoins to the transfer fluid (5) in the housing (1) with its side facing to the body (6) and which rests with the other side on an insulating film (24), resting on a spirally wound flat coil (25), that is supported by a coil support (26) and that can be supplied with high-voltage pulses out of a high voltage pulse generator (27).

17. Pulse wave generator according to one of the preceding claims, **characterized in that**
the pressure field generator (2) generates pressures of at least 0.6 up to 6 dyn/mm².

18. Pulse wave generator according to claim 16,
**characterized in that**
the amperage of the high voltage pulse is greater than 1 kA and the voltage is larger than 1 kV.

19. Pulse wave generator according to one of the preceding claims, **characterized in that**
an apparatus is integrated for the infusion of liquid active ingredient.

20. Pulse wave generator according to one of the preceding claims, **characterized in that**
it can be implanted.

21. Pulse wave generator according to one of the preceding claims, **characterized in that**
in the controller (3) is adjustable, that each pulse of the field generators (2) and/or (4) has a duration of max 0.1 sec.

22. Pulse wave generator according to one of the preceding claims, **characterized in that**
in the controller (3) is adjustable that up to 10,000 individual pulses of the field generators (2) and/or (4) at intervals of at most 0.5 sec act to the focal point (F) of the body (6), which is to be treated and that this series of pulses are repeated at an interval of one day up to one week over a period of up to 3 months.

23. Pulse wave generator according to one of the preceding claims, **characterized in that**
in the controller is adjustable that each pulse is extended to a series of pulses of up to 0.1 sec duration.

24. Pulse wave generator according to one of the preceding claims, **characterized in that**
the location of the focusing point (F) and/or the intensity of each field pulse and/or the timing of each field pulse can be set to the treatment of tumors and thrombi and other discomfort or pain sensations, which are localized in a limited space in or on the body (6) of living organisms.

25. Pulse wave generator according to one of the preceding claims, **characterized in that**
the location of the focusing point (F) and/or the intensity of each field pulse and/or the timing of each field pulse can be set to the treatment of tumors in the breast or in the pancreas or in the brain or in the prostate or in another organ or area or Creutzfeldt-Jacob disease or apoplectic stroke or cardiac arrhythmia or heart attack or interstitium or embolism or thrombosis or disorders of wound and bone healing or cerebral circulatory disorders or cerebral metabolic disorders such as Alzheimer's disease or Prostatic hypertrophy or hypertrophy and metaplasia of other organs or inflammation of organs or body structures or healing of chronic inflammatory diseases such as multiple sclerosis or macular degeneration or indigestion or wrinkling of the skin or endocrine diseases of the kidney or liver or diabetes.

26. Pulse wave generator according to one of the preceding claims, **characterized in that**
the location of the focusing point (F) and/or the intensity of each field pulse and/or the timing of each field pulse is adjustable for the purpose of an indirect treatment of an internal organ to the respectively associated celiac ganglion inferior or cervical inferior or cervical superior or sacral or pelvinum or on the respectively associated Head's skin District.

27. Pulse wave generator according to one of the preceding claims, **characterized in that**
the location of the focusing point (F) and/or the intensity of each field pulse and/or the timing of each field pulse is adjustable for the treatment of pain caused by tumors, rheumatoid diseases and their treatment or tensions or circulation problems such as in the legs as a result of metabolic disorders or heart attack and stroke or vasospastic diseases or internal diseases such as Whipple's disease, angina pectoris, stomach pain or functional abdominal pain or neurological and psychiatric diseases such as neuralgia, multiple sclerosis or neurovegetative syndrome.

28. Pulse wave generator according to one of the preceding claims, **characterized in that**
the location of the focusing point (F) and/or the intensity of each field pulse and/or the timing of each field pulse is adjustable for the modulation of the mitochondrial or cellular or extracellular metabolism or for the modulation of the cell matrix.

## Revendications

1. Générateur d'ondes d'impulsions, comprenant un boîtier 1 avec
- un générateur de champ de pression (2), duquel des impulsions de pression pouvant être commandées individuellement peuvent être émises et
- une unité de focalisation d'impulsions de pression (21) au moyen de laquelle les impulsions de pression peuvent être focalisées en un point de focalisation F dans ou sur un corps (6) d'un organisme et
- un coupleur d'impulsions de pression (22) duquel les d'impulsions de pression peuvent être transmises au corps (6) et
- une commande (3) pour l'intensité et l'instant de chaque impulsion de pression, sachant
qu'au moins un générateur de champ supplémentaire (4) est intégré, par lequel
- des impulsions de champ pouvant être commandées individuellement peuvent être produites dans le point de focalisation (F) et
- l'intensité et l'instant de chaque impulsion de pression peuvent être commandées,
**caractérisé en ce**
**qu'**une bobine sert de générateur de champ (4) pour produire un champ magnétique, laquelle est traversée, par la décharge d'un condensateur chargé jusqu'à 50 kV
- par le biais d'une chambre à plasma ou
- par le biais d'un éclateur
par une impulsion de courant très élevée jusqu'à 3 kA,
ce qui produit une impulsion électromagnétique
- dont la teneur en énergie est brièvement très élevée et
- qui, par induction, élève brièvement le potentiel transmembranaire d'une cellule tumorale ou d'une autre cellule fortement excitée, à une valeur normale.

2. Générateur d'ondes d'impulsions selon la revendication 1,
**caractérisé en ce que** le potentiel transmembranaire d'une cellule tumorale de 15 à 20 mV peut être élevé brièvement au potentiel transmembranaire d'une cellule saine d'environ 80 à 100 mV.

3. Générateur d'ondes d'impulsions selon la revendication 1,
**caractérisé en ce que** le potentiel transmembranaire d'une cellule fortement excitée par une douleur chronique d'environ 60 à 80 mV peut être élevé brièvement au potentiel transmembranaire d'une cellule saine d'environ 80 à 100 mV.

4. Générateur d'ondes d'impulsions, selon l'une des revendications précédentes, **caractérisé en ce que**
de chacun des générateurs de champ supplémentaires (4)
- un champ magnétique ou
- électrique ou
- électromagnétique
peut être produit.

5. Générateur d'ondes d'impulsions, selon l'une des revendications précédentes, **caractérisé en ce que** d'au moins un des générateurs de champ (2) ou (4), un champ alternatif peut être produit, dont
- l'amplitude et
- la fréquence et
- la durée d'activation et
- l'instant d'activation
peuvent être commandés.

6. Générateur d'ondes d'impulsions, selon la revendication précédente 3, **caractérisé en ce que** l'amplitude et/ou la fréquence
- augmente et/ou
- baisse et/ou
- oscille
pendant toute la durée d'activation.

7. Générateur d'ondes d'impulsions, selon l'une des revendications précédentes, **caractérisé en ce qu'un** transducteur (71) d'un appareil de diagnostic par ultrasons (7) est disposé dans l'axe longitudinal du générateur de champ de pression (2),
- qui fait saillie, à travers l'unité de focalisation d'impulsions de pression (21) et le coupleur d'impulsions de pression (22), quelque peu au-dessus de la surface extérieure du coupleur d'impulsions de pression (22) et
- peut être quelque peu rentré par pression dans le corps (6) et
- suit le plan de mesure du transducteur (71) à travers le point de focalisation (F).

8. Générateur d'ondes d'impulsions, selon l'une des revendications précédentes, **caractérisé en ce que** le générateur de champ de pression (2) est une membrane élastique durable,
- qui est au moins tendue en plusieurs points de son pourtour et
- peut être déviée en son centre ou près de celui-ci par une impulsion mécanique, qui est alignée
- environ verticalement à la surface de la membrane et
- sur le point de focalisation (F).

9. Générateur d'ondes d'impulsions, selon la revendication 8, **caractérisé en ce que** l'impulsion mécanique est produite par
- un électroaimant ou
- un cristal piézoélectrique ou
- un ressort précontraint ou
- un accumulateur de pression pneumatique ou hydraulique comprenant un cylindre ou
- l'allumage d'un agent moteur ou
- la bascule d'une came.

10. Générateur d'ondes d'impulsions, selon l'une des revendications précédentes, **caractérisé en ce que** le générateur de champ de pression (2) est une masse mobile dans le sens du point de focalisation (F) qui est déviée dans le sens du point de focalisation (F) par une impulsion mécanique.

11. Générateur d'ondes d'impulsions, selon la revendication 10, **caractérisé en ce que**
la masse mobile peut dégager
- une énergie cinétique d'au moins 5 millijoules
et
- permet d'atteindre une vitesse d'au moins 3 m/s et
la masse du corps solide peut être inférieure à 0,1 g.

12. Générateur d'ondes d'impulsions, selon l'une des revendications précédentes 10 ou 11, **caractérisé en ce qu'un** creux ou un évidement sur le côté de la masse mobile orienté vers le point de focalisation F sert d'unité de focalisation d'impulsions de pression.

13. Générateur d'ondes d'impulsions, selon la revendication précédente 12, **caractérisé en ce qu'un** canal d'air sort du creux ou de l'évidement et va jusqu'à un côté de la masse mobile détourné du corps (6).

14. Générateur d'ondes d'impulsions, selon l'une des revendications précédentes, **caractérisé en ce que** le coupleur d'impulsions de pression (22) est composé d'un sachet durablement flexible et rempli d'un liquide de transfert (5) qui est délimité
- sur le corps (6) par un côté et
- sur l'unité de focalisation d'impulsions de pression (21) par le côté opposé.

15. Générateur d'ondes d'impulsions, selon l'une des revendications précédentes, **caractérisé en ce que**
l'unité de focalisation d'impulsions de pression (21) est une lentille acoustique qui est composée d'un matériau dur et résistant aux vibrations ayant la forme d'une rondelle,
- dont le bord dans le boîtier (1) est ainsi tendu qu'elle est alignée transversalement au sens de l'impulsion de pression et
- présente un évidement concave sur au moins un côté qui est formé en symétrie de rotation par rapport au sens l'impulsion de pression et
- a sur les deux côtés respectivement, un contact sur toute la surface avec un liquide de transfert (5),
- qui remplit d'un côté le coupleur d'impulsions de pression (22) et
- est délimité sur le second côté sur le générateur de champ de pression (2) et
les surfaces de la rondelle sont ainsi formées qu'elles lient chaque partie d'une impulsion de pression du générateur de champ de pression (2) dans le point de focalisation (F).

16. Générateur d'ondes d'impulsions, selon l'une des revendications précédentes, **caractérisé en ce que** le générateur de champ de pression (2) est composé d'une membrane plane (23),
- qui contient du matériau conducteur d'électricité, par exemple du cuivre ou de l'aluminium et
- est délimitée par le côté orienté vers le corps (6) sur le liquide de transfert (5) se trouvant dans le boîtier (1) et
- repose par l'autre côté sur un film isolant (24),
- qui repose sur une bobine plate (25) enroulée en spirale,
- qui est tenue par un support de bobine (26) et
- peut être alimentée en impulsions haute tension par un générateur d'impulsions haute tension (27).

17. Générateur d'ondes d'impulsions, selon l'une des revendications précédentes, **caractérisé en ce que** le générateur de champ de pression (2) produit des pressions d'au moins 0,6 à 6 dyn/mm².

18. Générateur d'ondes d'impulsions, selon la revendication 16, **caractérisé en ce que** l'intensité de courant de l'impulsion haute tension est supérieure à 1 kA et la tension est supérieure à 1 kV.

19. Générateur d'ondes d'impulsions, selon l'une des revendications précédentes, **caractérisé en ce qu'un** appareil de perfusion de substances liquides est intégré.

20. Générateur d'ondes d'impulsions, selon l'une des revendications précédentes, **caractérisé en ce qu'il** peut être implanté.

21. Générateur d'ondes d'impulsions, selon l'une des revendications précédentes, **caractérisé en ce que** l'on peut régler dans la commande (3), que chaque impulsion individuelle des générateurs de champ (2) et/ou (4) ait une durée de max 0,1 sec.

22. Générateur d'ondes d'impulsions, selon l'une des revendications précédentes, **caractérisé en ce que** l'on peut régler dans la commande (3), que
- jusqu'à 10.000 impulsions individuelles des générateurs de champ (2) et/ou (4) à distance de max. 0,5 sec agissent sur le point de focalisation (F) à traiter du corps et
- ces séries d'impulsions soient répétées à distance d'un jour à une semaine sur une durée allant jusqu'à (3) mois.

23. Générateur d'ondes d'impulsions, selon l'une des revendications précédentes, **caractérisé en ce que** l'on peut régler dans la commande, que chaque impulsion individuelle soit élargie à une série d'impulsions d'une durée allant jusqu'à 0,1 sec.

24. Générateur d'ondes d'impulsions, selon l'une des revendications précédentes, **caractérisé en ce que** l'emplacement du point de focalisation (F) et/ou l'intensité et/ou l'instant de chaque impulsion de champ peu(ven)t être ajusté(e)(s) au traitement de tumeurs et de thromboses et autres gênes ou sensations de douleur localisées dans un espace délimité dans ou sur le corps (6) d'organismes.

25. Générateur d'ondes d'impulsions, selon l'une des revendications précédentes,
**caractérisé en ce que**
- l'emplacement du point de focalisation (F) et/ou
- l'intensité de chaque impulsion de champ et/ou
- l'instant de chaque impulsion de champ peu(ven)t être ajusté(e)(s) au traitement
- de tumeurs dans la poitrine ou dans le pancréas ou dans le cerveau ou dans la prostate ou dans un autre organe ou une autre zone ou
- de maladie de Creutzfeldt-Jacob ou
- d'apoplexie ou
- de troubles du rythme cardiaque ou
- d'infarctus du myocarde ou
- d'interstitium ou
- d'embolie ou
- de thrombose ou
- de troubles de la guérison osseuse et de blessures ou
- de troubles sanguins cérébraux ou
- de troubles métaboliques cérébraux comme la maladie d'Alzheimer ou
- d'hypertrophies de la prostate ou
- d'hypertrophies et métaplasies d'autres organes ou
- d'inflammations d'organes ou de structures corporelles ou
- de guérison d'inflammations chroniques comme la sclérose en plaques ou
- de dégénération maculaire ou
- de troubles de la digestion ou
- de formation de rides de la peau ou
- de maladies endocriniennes des reins ou du foie ou
- de diabète.

26. Générateur d'ondes d'impulsions, selon l'une des revendications précédentes, **caractérisé en ce que**
- l'emplacement du point de focalisation (F) et/ou
- l'intensité de chaque impulsion de champ et/ou
- l'instant de chaque impulsion de champ
peu(ven)t être ajusté(e)(s) pour le traitement indirect d'un organe interne sur
- le ganglion .coeliacum inferior ou cervicale inferior ou cervicale superior ou sacrale ou pelvinum respectif correspondant ou
- sur l'échantillon de peau de Head correspondant.

27. Générateur d'ondes d'impulsions, selon l'une des revendications précédentes, **caractérisé en ce que**
- l'emplacement du point de focalisation (F) et/ou
- l'intensité de chaque impulsion de champ et/ou
- l'instant de chaque impulsion de champ
peu(ven)t être ajusté(e)(s) au traitement de douleurs à la suite
- de tumeurs, maladies rhumatoïdes et leur traitement ou
- de tensions ou
- de troubles de la circulation sanguine par ex. des jambes à la suite de maladies métaboliques ou
- d'infarctus du myocarde et d'accident vasculaire cérébral ou
- de maladies vasospastiques ou
- de maladies internes comme la maladie de Whipple, angina pectoris, de douleurs stomacales ou de troubles abdominaux fonctionnels ou
- de maladies neurologiques et psychiatriques comme des névralgies, la sclérose en plaques, le syndrome neurovégétatif.

28. Générateur d'ondes d'impulsions, selon l'une des revendications précédentes, **caractérisé en ce que**
- l'emplacement du point de focalisation (F) et/ou
- l'intensité de chaque impulsion de champ et/ou
- l'instant de chaque impulsion de champ
peu(ven)t être ajusté(e)(s) à la modulation du
- métabolisme mitochondrial ou
- cellulaire ou
- extracellulaire ou de la matrice cellulaire.
